# FASCICULE DE BREVET EUROPEEN

(11) **EP 1 409 539 B1**
(45) Date de publication et mention de la délivrance du brevet: **29.03.2006**
(21) Numéro de dépôt: 02764984.7
(22) Date de dépôt: 12.07.2002
(51) Int. Cl.: C07K 14/495, A23J 1/20, A61K 38/18

(54) **PROCEDE D'OBTENTION D'UNE FRACTION PROTEIQUE ENRICHIE EN TGF-BETA SOUS FORME ACTIVEE, FRACTION PROTEIQUE ET APPLICATIONS THERAPEUTIQUES**
VERFAHREN ZUR HERSTELLUNG EINER PROTEINHALTIGEN FRAKTION, MIT BETA-TGF IN AKTIVIERTER FORM BEREICHERT, SOWIE DIE SO ERHALTENE FRAKTION UND DEREN THERAPEUTISCHE VERWENDUNGEN
METHOD FOR OBTAINING A TGF-BETA ENRICHED PROTEIN FRACTION IN ACTIVATED FORM, PROTEIN FRACTION AND THERAPEUTIC APPLICATIONS

(30) Priorité: 13.07.2001 FR 0109390
(43) Date de publication de la demande: 21.04.2004
(73) Titulaire: Pierre Jouan Biotechnologies S.A., 35000 Rennes (FR)
(72) Inventeur: MAUBOIS, Jean-Louis, F-35740 Pace (FR); FAUQUANT, Jacques, F-35160 Pleumeleuc (FR); JOUAN, Pierre, F-35510 Cesson Sevigne (FR); BOURTOURAULT, Michel, F-35230 Noyal Châtillon sur Seiche (FR)
(74) Mandataire: Catherine, Alain
(86) Numéro de dépôt international: PCT/FR2002/002489
(87) Numéro de publication internationale: WO 2003/006500

(56) Documents cités:
- WO-A-01/25276
- M I ROGERS ET AL.: "Transforming growth factor beta in bovine milk. concentration, stability and molecular mass form" JOURNAL OF ENDOCRINOLOGY, vol. 151, 1996, pages 77-86, XP000644717 BRISTOL, GB ISSN: 0022-0795
- DATABASE BIOSIS [en ligne] BIOSCIENCES INFORMATION SERVICE, PHILADELPHIA, PA, US; XU, RUO-JUN (1) ET AL: "Detection and characterisation of transforming growth factor-beta in porcine colostrum." retrieved from STN Database accession no. PREV199900054071 XP002198382 & BIOLOGY OF THE NEONATE, ( JAN., 1999 ) VOL. 75, NO. 1, PP. 59-64.,
- DATABASE BIOSIS [en ligne] BIOSCIENCES INFORMATION SERVICE, PHILADELPHIA, PA, US; JIN Y ET AL: "SEPARATION PURIFICATION AND SEQUENCE IDENTIFICATION OF TGF-BETA-1 AND TGF-BETA-2 FROM BOVINE MILK." retrieved from STN Database accession no. PREV199293015546 XP002198383 & J PROTEIN CHEM, (1991) 10 (5), 565-575.,

## Description

La présente invention se rapporte au domaine de la purification du facteur TGF-β à partir d'une matière première laitière.

### ART ANTERIEUR

Les laits, en particulier le lait humain et le lait de toutes les femelles de mammifères, contiennent de nombreux polypeptides biologiquement actifs, notamment de nombreux facteurs de croissance. L'un des facteurs de croissance contenus dans le lait est le TGF-β (pour « Transforming Growth Factor bêta).

Le vocable TGF-β désigne une famille comprenant divers facteurs de croissance. Les TGF-β1 et TGF-β2 sont les deux formes homologues du TGF-β. Ce sont des constituants homodimériques constitués de deux chaînes peptidiques de 112 acides aminés chacune, identiques et reliées entre elles par l'intermédiaire d'un pont disulfure interchaîne. Leur poids moléculaire est de 25.000 Daltons. Les polypeptides TGF-β1 et TGF-β2 présentent 72% d'homologie structurale et leurs propriétés biologiques sont identiques. Il existe une identité totale dans la séquence d'acides aminés des TGF-β2 d'origine humaine et bovine.

Les TGF-β peuvent être obtenus par recombinaison génétique sous forme purifiée. Mais les préparations de TGF β recombinants sont susceptibles de contenir une faible part de protéines bactériennes dont certaines sont allergisantes. De plus, par définition, des préparations purifiées de TGF-β recombinants ne contiennent aucune protéine associée de lait, qui serait susceptible de compléter ou de potentialiser l'effet biologique du TGF-β

Le lait de vache contient à la fois du TGF-β1 et du TGF-β2. Le TGF-β2 est majoritaire, et représente 90% en poids du TGF-β retrouvé dans le lait, le TGF-β1 quant à lui, représente 10% en poids du contenu total en TGF-β du lait.

Dans le lait, plus de 90% du TGF-β2 se retrouvent sous une forme latente, c'est-à-dire sous une forme non biologiquement active.

Diverses études académiques ont montré que le taux de TGF-β2 dans le lait était de 12 à 150 µg/l dans le colostrum de 3,7 à 3,8 µg/l dans le lait cru et dans le lait pasteurisé, 4,3 µg/l dans le lait écrémé et 3,7µg/l dans le lactosérum.

Le TGF-β possède un large spectre d'activité biologique conférant à ce polypeptide un grand intérêt thérapeutique, dans la prévention ou dans le traitement d'une grande variété d'affections ou de pathologies.

Le TGF-β est biologiquement actif sur la matrice extra-cellulaire. Le TGF-β stimule la synthèse des protéines matricielles et augmente la synthèse du collagène et de la fibronectine dans les fibroblastes. Il inhibe aussi la synthèse d'enzymes protéolytiques telles que la collagénase et les métalloprotéases. Le TGF-β augmente la sécrétion d'inhibiteurs des protéases tels que l'inhibiteur de l'activateur du plasminogène ou encore les inhibiteurs de métalloprotéases.

Le TGF-β exerce également une activité biologique sur le squelette. En particulier, le TGF-β est présent, en fortes concentrations, dans l'os. Il joue un rôle dans la formation du cartilage et stimule la résorption des ostéoclastes, ainsi que l'activation des ostéoblastes. Il agit comme un inhibiteur naturel de la résorption osseuse et comme un stimulant de la formation osseuse.

Le TGF-β est également actif sur les lymphocytes. A titre illustratif, le TGF-β inhibe la prolifération des lymphocytes T et favorise l'activité des cellules tueuses dites « Natural Killers ».

Le TGF-β est également un puissant agent anti-inflammatoire. Il diminue la production de cytokines pro-inflammatoires. Il possède des propriétés immunosuppressives et inhibe la prolifération des lymphocytes T activés.

En outre, le TGF-β possède des effets anti-prolifératifs. Il est un inhibiteur puissant des cellules épithéliales. Il exerce aussi une forte activité anti-mitogénique vis-à-vis des cellules mésenchymateuses, des fibroblastes embryonnaires, des cellules endothéliales ainsi que des lymphocytes T et B. Il est aussi un inhibiteur puissant de la croissance des hépatocytes et il pourrait jouer un rôle important dans le maintien de la quiescence de ceux-ci. Le TGF-β agit aussi comme un facteur de régulation négative de l'épithélium mammaire.

Le TGF-β possède également des effets anti-cancéreux. Durant la carcinogenèse, les cellules cancéreuses peuvent perdre leur aptitude à répondre au TGF-β. Néanmoins, certaines tumeurs à cellules épithéliales sont sensibles aux effets anti-prolifératifs du TGF-β. Tel est le cas des cellules du cancer du sein.

Par ailleurs, le TGF-β agit sur la prolifération et la différenciation des cellules leucémiques. Il inhibe la prolifération des cellules promyélocytaires. Il pourrait agir en synergie avec l'acide rétinoïque et la vitamine D3.

La demande de brevet européen n° EP 0 527.283 au nom de Société des Produits NESTLE S.A. décrit un procédé de préparation d'un produit dérivé du lait contenant du TGF-β, dans lequel un lait entier est écrémé par centrifugation, dessalé sur une colonne PD-10 (Pharmacia) puis stérilisé par filtration sur une membrane « Millipore » d'un diamètre de pore égal à 0,2 µm. Puis le lait écrémé est stérilisé et ajusté à pH 4 à l'aide d'une solution de HCl 1N, puis centrifugé à 40.000 g pendant 60 minutes afin de séparer la caséine précipitée du lactosérum. Le lactosérum ainsi séparé est neutralisé par la soude 1 N, puis dialysé. Toutefois, ce procédé qui permet d'éliminer la caséine du lait écrémé de départ, n'est pas un procédé de purification du TGF-β. Il conduit, en effet, à un produit final contenant la totalité des protéines du lactosérum initial où est présent le TGF-β, mais sans enrichissement significatif de la teneur en cette molécule.

Dans l'état de la technique, plusieurs procédés d'obtention, à partir de lait, de fractions protéiques enrichies en TGF-β ont été décrits.

La demande de brevet européen n° EP 0 313 515 au nom de CIBA GEIGY décrit un procédé de purification d'un facteur de croissance contenu dans le lait, mettant en oeuvre des étapes successives de chromatographie, en utilisant notamment des résines échangeuses de cations, des supports pour chromatographie d'interaction hydrophobe (HPLC-RP) ou encore des supports chromatographiques d'exclusion par la taille.

La demande PCT N°WO 01 25.276 au nom de CAMPINA MELKUNIE B.V. décrit un procédé d'extraction de TGF-β et de facteurs de croissance analogues à l'insuline (IGF-1) à partir d'un produit laitier. Le procédé comporte les étapes consistant à :
a) récupérer une fraction de base du produit laitier par chromatographie à échange de cation;
b) faire passer la fraction obtenue à l'étape a) sur une colonne d'hydroxyapatite;
c) éluer la colonne d'hydroxyapatite à l'aide d'éluants appropriés de manière à obtenir notamment une fraction contenant du TGF-β sensiblement exempte d'IGF-1.

Les procédés décrits ci-dessus présentent tous des inconvénients techniques. En effet, les procédés de purification du TGF-β mettant en oeuvre des étapes successives de chromatographie sont longs et fastidieux. De plus, le nombre important d'étapes chromatographiques nécessaires à l'obtention d'un degré de purification désiré réduit considérablement le rendement final, du fait de la dégradation progressive du TGF-β au cours du temps de purification, et de la perte inévitable de TGF-β biologiquement actif à chacune des étapes chromatographiques. Outre l'utilisation de solutions salines et de solutions de régénération à haut pouvoir polluant qu'il faut ensuite éliminer, ces procédés présentent un risque élevé de contamination bactérienne du produit final.

Il existe donc un besoin dans l'état de la technique de procédés améliorés de purification du TGF-β à partir d'un produit laitier, qui ne comporte pas les inconvénients décrits ci-dessus pour les procédés de l'art antérieur.

### SOMMAIRE DE L'INVENTION

L'invention a pour objet un procédé d'obtention d'une fraction protéique fortement enrichie en TGF-β sous forme activée, à partir d'une solution liquide riche en protéines dites solubles de la phase aqueuse du lait et/ou du lactosérum, ledit procédé comprenant les étapes suivantes :
a) ajustement des protéines solubles purifiées à une concentration de 5 à 30 g/litre de solution.
b) précipitation d'une partie des protéines du lactosérum par traitement acide de la solution ainsi obtenue à un pH compris entre 4 et 5,5 et à une température comprise entre 55°C et 68°C;
c) microfiltration de la solution traitée avec diafiltration, afin d'obtenir respectivement un rétentat de microfiltration et un microfiltrat;
d) récupération du rétentat de microfiltration contenant la fraction protéique fortement enrichie en TGF-β.
e) séchage du rétentat de microfiltration, diafiltré, afin d'obtenir une poudre fortement enrichie en TGF-β.

De préférence, l'étape a) d'ajustement est réalisée par dilution d'une solution contenant les protéines solubles du lait, tel qu'un isolat de protéines de lactosérum ; également désigné « WPI ».

De préférence, l'étape b) de précipitation est réalisée à une température comprise entre 60°C et 68°C, et encore plus préférentiellement à environ 63°C.

Avantageusement, la durée de l'étape b) de précipitation est comprise entre 30 secondes et 10 minutes, de préférence entre 30 secondes et 5 minutes et de manière tout à fait préférée d'environ 2 minutes.

De manière préférée, la microfiltration de l'étape c) est réalisée sur une membrane de microfiltration ayant une taille moyenne de pores comprise entre 0,8 et 1,6 µm et possédant une distribution de taille de pore resserrée.

L'invention a également pour objet l'obtention d'une fraction protéique fortement enrichie en GF-β, susceptible d'être obtenue par le procédé ci-dessus, et qui a une teneur en TGFβ2 sans forme activée supérieure à 5 µg par gramme de protéines totales du lactoserum, les autres protéines du lactosérum étant majoritairement constituées d'α-lactalbumine.

Elle est également relative à une composition pharmaceutique comprenant une fraction protéique telle que définie ci-dessus; additionnée le cas échéant, d'un un ou plusieurs excipients physiologiquement compatibles.

Elle concerne aussi l'utilisation d'une fraction protéique telle que définie ci-dessus pour la préparation d'un médicament pour la prévention ou le traitement de diverses pathologies pour lesquelles le TGF-β constitue un composé d'intérêt thérapeutique.

### DESCRIPTION DE LA FIGURE UNIQUE

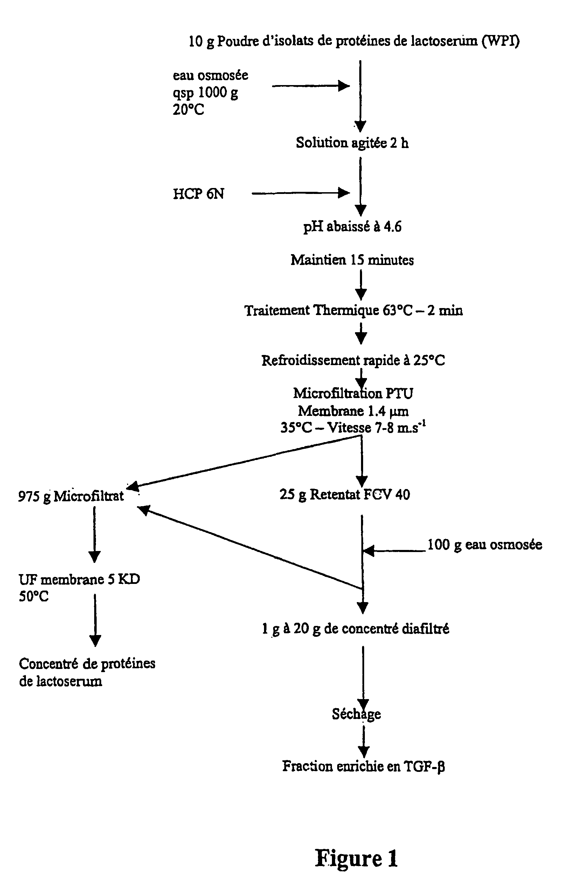

La figure 1 représente un schéma du procédé d'obtention d'une fraction protéique riche en TGβ de l'invention.

### DESCRIPTION DETAILLEE DE L'INVENTION

Le demandeur a mis au point un procédé à la fois simple et rapide d'obtention d'une fraction protéique fortement enrichie en TGF-β à partir d'une solution liquide riche en protéines du lactosérum.

Par « TGF-β », on entend une association ou un mélange de TGF-β comprenant au moins 90% en poids de TGF-β2 et au plus 10% en poids de TGF-β1.

Notamment, le procédé de l'invention comporte un faible nombre d'étapes de courte durée, ce qui permet d'éviter une perte significative de l'activité biologique du TGF-β due à une dégradation de cette protéine au cours du temps pendant le procédé de purification.

En outre, le procédé selon l'invention ne comporte aucune étape de chromatographie nécessitant l'adsorption successive des protéines d'intérêt sur plusieurs supports, puis leur désorption des supports par élution, qui entraînent obligatoirement une perte significative du TGF-β et donc des conséquences néfastes sur le rendement final du procédé.

L'invention concerne un procédé d'obtention d'une fraction protéique enrichie en TGF-β sous forme activée, à partir d'une solution liquide riche en protéines du lactosérum, ledit procédé comprenant les étapes suivantes :
a) ajustement des protéines purifiées de lactosérum à une concentration de 5 à 30 g/litre de solution.
b) précipitation d'une partie des protéines du lactosérum par traitement acide de la solution liquide à un pH compris entre 4 et 5,5 et à une température comprise entre 55°C et 68°C ;
c) microfiltration de la solution ainsi traitée avec diafiltration, afin d'obtenir respectivement un rétentat de microfiltration et un microfiltrat;
d) récupération du rétentat de microfiltration contenant la fraction protéique fortement enrichie en TGF-β.
e) séchage du rétentat diafiltré de microfiltration afin d'obtenir une poudre fortement enrichie en TGF-β.

La solution de départ riche en protéines du lactosérum peut être de toute nature. De préférence, il s'agit d'un isolat de protéines du lactosérum (aussi désigné WPI pour « Whey proteins isolate »), qui peut être préparé par toute méthode connue de l'homme du métier.

La solution riche en protéines du lactosérum a, de préférence, la composition suivante :
Matière azotée totale : 10 g/Kg.
Extrait sec: 10,4 g/Kg.
Soit 96 % de protéines dans l'extrait sec.

### Etape a) du procédé

L'étape a) du procédé consiste préférentiellement en une dilution des protéines par de l'eau osmosée pour obtenir une concentration de 5 à 30 g de protéines par kilogramme de solution.

### Etape b) du procédé

L'étape b) du procédé consiste en une étape de précipitation fractionnée des protéines du lactosérum dans des conditions opératoires permettant une précipitation sélective de la fraction protéique contenant la quasi-totalité du TGF-β initialement contenu dans la solution liquide riche en protéines du lactosérum, par exemple un WPI.

Cet objectif a été atteint par le demandeur en combinant certains paramètres de pH et de température.

On a trouvé, selon l'invention, que l'application d'un seul des paramètres, soit le pH, soit la température, ne permet pas d'atteindre le degré désiré de précipitation des protéines.

Lorsque l'étape b) est réalisée à une température inférieure à 55°C, la précipitation de la fraction protéique riche en TGF-β n'est pas obtenue. De même, lorsque la température de traitement est supérieure à 68°C, et pour des températures croissantes, la co-précipitation, avec le TGF-β, d'une fraction de plus en plus grande des protéines de lactosérum est observée. Ainsi, pour des températures supérieures à 70°C, la quasi-totalité des protéines de lactosérum, contenues initialement dans la solution initiale est précipitée et l'enrichissement sélectif en TGF-β n'est plus obtenu.

A titre illustratif, pour un pH à 4,6, les résultats de précipitation observés à des températures croissantes sont présentés dans le tableau 1 ci-dessous.

Le tableau 1 ci-dessous résume l'évolution de la précipitation du TGF-β et des protéines du lactosérum à pH 4,6 en fonction de l'intensité du traitement thermique (durée du traitement : 2 minutes.

**TABLEAU 1**

| Température (°C) | % du TGF-β initial^{a} | % des autres protéines précipitées^{b} |
|---|---|---|
| 25°C | 40% | 10% |
| 50°C | 43% | 12% |
| 60°C | 67% | 15% |
| 63°C | 84% | 16% |
| 65°C | 94% | 30% |
| 70°C | 100% | 50% |

| | | |
|---|---|---|
| ^{a} Pourcentage du TGF-β contenu initialement dans la solution liquide riche en protéines du lactosérum (WPI) qui est retrouvé dans la fraction protéique précipitée. | | |
| ^{b} Pourcentage des protéines totales contenues initialement dans la solution liquide riche en protéines du lactosérum (WPI) qui est retrouvé dans la fraction protéique précipitée. | | |

Les résultats du tableau 1 montrent qu'au pH 4,6, et à une température de 50°C, seuls 43% du TGF-β contenu initialement dans la solution riche en protéines du lactosérum sont précipités. A la température de 70°C, 100% du TGF-β2 initial sont précipités, mais la précipitation est peu sélective du fait que 50% des protéines totales initialement contenues dans la solution initiale de protéines du lactosérum co-précipitent avec le TGF-β, ce qui ne permet pas d'atteindre le degré désiré d'enrichissement en TGF-β.

De préférence, l'étape b) du procédé selon l'invention est réalisée à un pH compris entre 4 et 5,5, et encore plus avantageusement à un pH compris entre 4 et 5. De manière tout à fait préférée, l'étape b) du procédé est réalisée à un pH compris entre 4,3 et 4,9, encore mieux entre 4,4 et 4,8, préférentiellement entre 4,5 et 4,7. Le pH optimal de mise en oeuvre est d'environ 4,6.

Préférentiellement, le pH est ajusté à l'aide d'un acide concentré afin de réduire au maximum les phénomènes de dilution découlant de l'ajout d'un volume de liquide supplémentaire. De préférence, on utilise du HCl concentré et de manière tout à fait préférée du HCl à la concentration 6N, notamment du fait de sa manutention plus facile et moins dangereuse que pour d'autres acides concentrés.

La température de mise en oeuvre de l'étape b) du procédé est avantageusement comprise entre 55°C et 68°C, de préférence entre 61 °C et 65°C, de manière préférée entre 62°C et 64°C et de manière tout à fait préférée à environ 63°C.

Sans vouloir être lié par une quelconque théorie, le demandeur pense que le TGF-β, qui se trouve essentiellement sous forme latente dans la solution initiale de protéines du lactosérum, est converti en sa forme activée (biologiquement active) lors de l'étape b) du procédé.

La combinaison des paramètres de température et de pH ci-dessus rend possible la mise en oeuvre de l'étape de précipitation pendant une durée courte, ce qui permet d'éviter la dégradation du TGF-β. Ainsi, la durée de l'étape b) de précipitation est comprise entre 30 secondes et 10 minutes, de préférence entre 30 secondes et 5 minutes, encore mieux entre 1 minute et 3 minutes, et de manière tout à fait préférée d'environ 2 minutes.

Lorsque l'étape b) du procédé est réalisée pendant 15 minutes à pH 7,0 à 80 °C, une dénaturation du TGF-β est observée, cette dénaturation étant croissante avec la durée de l'étape de précipitation au-delà de 15 minutes.

Le traitement thermique peut être de toute nature. Il est préférentiellement réalisé à l'aide d'échangeurs thermiques de type tubulaire ou encore d'échangeurs thermiques à surface raclée connus de l'homme de métier pour le traitement thermique de solutions visqueuses ou de suspensions telles que les fromages frais.

Selon un mode de réalisation particulier de l'étape b) du procédé, la solution précipitée de TGF-β est refroidie rapidement.

Le demandeur a, en effet, observé qu'un refroidissement rapide après précipitation influe positivement sur la texture du précipité obtenu, et favorise en conséquence la mise en oeuvre des étapes ultérieures du procédé, et plus particulièrement l'étape de microfiltration, le précipité ne devant pas être trop fin pour ne pas colmater la membrane.

De préférence, le refroidissement est effectué dans un échangeur thermique à plaques ou tubulaire, avec de l'eau à température ambiante, de préférence entre 20 et 30°C, avantageusement à environ 25°C.

La durée de l'étape de refroidissement est préférentiellement comprise entre 1 et 20 minutes, et de manière tout à fait préférée entre 2 et 10 minutes.

Le produit final de l'étape b) est donc un liquide contenant une suspension de protéines précipitées incluant le TGF-β dans une solution de protéines non précipitées du lactosérum.

### Etape c) du procédé

La suspension de protéines contenant le TGF-β dans une solution de protéines non précipitées du lactosérum obtenue à l'étape b) est microfiltrée avec diafiltration.

Le précipité est soumis à une étape de microfiltration avec diafiltration afin d'éliminer la majorité des protéines solubles restantes du lactosérum tout en concentrant la fraction protéique d'intérêt contenant majoritairement le TGF-β.

Afin d'éviter des pertes significatives en TGF-β tout en éliminant une majorité des protéines solubles du lactosérum non désirées, la microfiltration est réalisée avec une membrane dont la distribution gaussienne de taille de pores est resserrée.

La microfiltration est réalisée de préférence avec une membrane de microfiltration ayant une taille moyenne de pores comprise entre 0,8 et 1,6 µm et possédant une distribution gaussienne de taille de pores resserrée.

De manière tout à fait préférée, la microfiltration avec diafiltration est réalisée dans des conditions de pression transmembranaire uniforme sur la totalité de la surface de la membrane filtrante.

La recirculation à co-courant du perméat représente un premier mode de réalisation de l'étape de microfiltration du procédé selon l'invention permettant l'obtention d'une pression transmembranaire substantiellement uniforme sur l'ensemble de la surface de la membrane du filtre.

Ainsi, la recirculation à co-courant du perméat à la surface externe du support de la membrane filtrante permet l'obtention d'une perte de charge (différence entre la pression d'entrée et la pression de sortie des fluides circulant de chaque côté de la membrane filtrante) identique dans chacun des compartiments du filtre et donc une différence de pression substantiellement identique en chaque point donné de la membrane filtrante, et ceci sur la totalité de la surface filtrante. La technique de recirculation du perméat à co-courant est par exemple décrite dans le brevet suédois n° SW 74 16 257 (Sandblom).

Lorsqu'on pratique la recirculation du perméat à co-courant et qu'on désire assurer une pression transmembranaire uniforme, on a obtenu les meilleurs résultats avec des membranes minérales ou céramiques, telles que celles en alumine alpha, mises sur le marché par la Société des Céramiques Techniques (France), sous la marque Membralox, ou par la Société Orelis France sous la marque KERASEP, ou encore les membranes de marque STERILOX .

Selon un autre aspect, la membrane filtrante est placée sur un support macroporeux à gradient longitudinal de perméabilité. Un tel support possède une constitution telle qu'il comporte un gradient de porosité qui décroît d'une extrémité à l'autre de la membrane filtrante.

Grâce à un tel support de filtre, la résistance hydraulique décroît d'une extrémité à l'autre de la membrane filtrante, ce qui permet l'obtention d'une pression transmembranaire uniforme, tout au long du chemin membranaire.

Un tel type de filtre est avantageusement réalisé à partir de céramique, tel que le support de filtre décrit dans la demande de brevet français n° FR 97 04 359.

Selon encore un autre aspect, on peut aussi réaliser une filtration membranaire dynamique, telle que décrite dans le brevet français 93 06 321 (publication 2 692 441), par exemple en utilisant des membranes organiques.

Selon un tel mode de réalisation du procédé de l'invention, la membrane filtrante et son support sont placés sur un axe rotatif, ledit dispositif étant complété par la disposition d'un disque rotatif à faible distance de la membrane de microfiltration.

La rotation du disque placé à faible distance (environ 4 mm) de la membrane de microfiltration génère une contrainte de cisaillement de 50 à 100 fois plus élevée que lors d'une filtration tangentielle classique, cette contrainte de cisaillement agissant dans les trois dimensions ( radiale, tangentielle et axiale). Dans un tel dispositif, la génération de la contrainte de cisaillement à la paroi est découplée de la pression transmembranaire. De tels procédés de filtration membranaire dynamique sont également décrits dans les brevets US 5,037 532, 3,997,447 et 4,956,102.

De manière préférée, la membrane de microfiltration possède une taille moyenne de pores comprise entre 1 et 1,6 µm et de manière tout à fait préférée d'environ 1,4 µm, telle que celle commercialisée par la société EXEKIA sous la référence « Stérilox, 1,4 µm classique», ce qui requiert la mise en oeuvre dans l'équipement de microfiltration de la recirculation à co-courant

du microfiltrat afin d'obtenir une pression transmembranaire uniforme ou sous la référence « Stérilox, 1,4 µm GP ».

De préférence, la diafiltration est réalisée avec de l'eau osmosée ou de l'eau déminéralisée, filtrée stérilement.

Préférentiellement, la diafiltration est réalisée en mettant en oeuvre de 1 à 6 diavolumes, de préférence environ 4 diavolumes, afin d'obtenir une élimination maximale des protéines solubles du lactosérum autres que le TGF-β.

Préférentiellement, la microfiltration avec diafiltration est réalisée à un pH compris entre 4 et 5,5, de préférence entre 4,3 et 5 et de manière tout à fait préférée à un pH d'environ 4,6 ce qui permet de garder le TGF-β sous forme précipitée.

L'ajustement du pH est effectué avec un acide concentré, de préférence du HCl concentré, avantageusement à la concentration 6N.

Les produits finaux de l'étape de microfiltration avec diafiltration sont respectivement :
- d'une part un rétentat fortement enrichi en TGF-β; et
- d'autre part, un microfiltrat contenant des protéines solubles du lactosérum. Par exemple, ce microfiltrat contient 8,0 g/l desdites protéines solubles pour une teneur en matière sèche d'environ 8,8g/l. Ces protéines de lactosérum qui n'ont pas précipité peuvent avantageusement être concentrées à nouveau, par tout moyen connu de l'homme de l'art, ultrafiltration sur membrane, par exemple, et être utilisées, une fois concentrées, pour la plupart des applications conventionnelles des WPI.

### Récupération du rétentat de microfiltration

A l'issue de l'étape de microfiltration avec diafiltration, le rétentat de microfiltration est récupéré et constitue la fraction protéique fortement enrichie en TGF-β.

En général, le rétentat de microfiltration contient de 6,5 à 17 µg/l de TGF-β pour 1 g de protéines totales.

Avantageusement, le rétentat de microfiltration, après récupération, est séché, par tout moyen connu de l'homme de métier (lyophilisation, atomisation, etc...)en vue d'obtenir la fraction protéique fortement enrichie en TGF-β sous la forme d'une poudre.

La poudre de rétentat renferme environ 15% des protéines initiales du lactosérum de la solution de départ. L'ensemble des étapes du procédé selon l'invention a donc permis d'éliminer 85% des protéines du lactosérum contenues initialement dans la solution de départ. Dans cette poudre, le taux de TGF-β est généralement compris entre 6,5 et 17 µg/g de poudre, ce qui correspond approximativement à la récupération de 70% du TGF-β initialement contenu dans la solution de protéines du lactosérum de départ. La teneur en TGFβ2 par rapport au poids de la poudre correspond à une teneur de TGFβ2 de 6 µg à 15µg, par gramme de protéines totales contenues dans la poudre.

Cette fraction protéique fortement enrichie en TGF-β peut être utilisée telle quelle, le cas échéant en association avec un ou plusieurs excipients physiologiquement compatibles.

Cette fraction protéique fortement enrichie en TGF-β peut également être soumise à des étapes supplémentaires de purification afin d'aboutir à un produit final possédant une teneur encore plus grande en TGF-β (microfiltration, centrifugation, acidification, chromatographie).

Ces étapes supplémentaires de purification permettent l'élimination des protéines insolubles du lactosérum, qui sont constituées majoritairement d'α-lactalbumine et d'immunoglobulines.

### Mode de réalisation préféré pour la préparation de la solution riche en protéines du lactosérum de départ.

Selon un mode de réalisation avantageux de l'invention, la solution riche en protéines du lactosérum de départ est obtenue par la mise en oeuvre des étapes suivantes:
i) microfiltration tangentielle d'un lait écrémé sur une membrane ayant une taille moyenne de pores de 0,1 µm dans un équipement permettant l'obtention d'une pression transmembranaire uniforme, puis récupération du microfiltrat ;
ii) concentration du microfiltrat obtenu à l'étape i) par ultrafiltration avec diafiltration à l'aide d'une membrane ayant un pouvoir de coupure compris entre 5000 daltons et 20000 daltons, telle que celles couramment utilisées par l'homme de métier pour la préparation des concentrés de protéines, à partir de lactosérum ;
iii) récupération du rétentat d'ultrafiltration diafiltré ;
iv) facultativement, dilution des protéines contenues dans le rétentat d'ultrafiltration diafiltré afin d'obtenir la solution de protéines du lactosérum de départ, telle que décrite dans la présente invention.

De préférence, la microfiltration tangentielle est réalisée avec une membrane du type Membralox (constituée d'alumine seule ou d'un mélange alumine-zircone) 0,1 µm classique, ce qui requiert la mise en oeuvre dans l'équipement de microfiltration de la recirculation à co-courant du microfiltrat (concept hydraulique dit de la pression transmembranaire uniforme) ou membrane Membralox 0,1 µm de référence GP telles que celles commercialisées par la société EXEKIA.

Le rétentat obtenu à la suite de l'étape i) de microfiltration tangentielle contient majoritairement les caséines du lait sous forme micellaire.

Le microfiltrat obtenu à l'issue de l'étape i) contient essentiellement les protéines solubles du lait, le lactose, les formes azotées non-protéiques et les sels minéraux solubles.

Le microfiltrat de l'étape i) est soumis à une étape d'ultrafiltration avec diafiltration afin de concentrer les protéines du lactosérum. De préférence, l'ultrafiltration est réalisée avec des membranes ayant un seuil de coupure compris entre 1 et 20 kD, de préférence environ 5 kD.

La diafiltration est réalisée préférentiellement avec de l'eau osmosée, le volume de diafiltration étant compris entre 2 et 6 diavolumes, et de préférence environ 4 diavolumes.

L'ultrafiltrat contient l'azote soluble du lait (azote non protéique) ainsi que du lactose et des sels minéraux.

Par exemple, le rétentat d'ultrafiltration récupéré à l'étape iii) contient environ 200g de protéines totales par litre de rétentat.

Après récupération du rétentat, les protéines contenues dans ce dernier sont diluées afin d'obtenir la solution de protéines du lactosérum de départ. La dilution est préférentiellement réalisée avec de l'eau osmosée.

Le cas échéant, le rétentat d'ultrafiltration récupéré à l'étape iii contenant les protéines à la concentration de 200 g/l peut être séché sous forme de poudre avant son emploi comme matériau de départ du procédé d'obtention d'une fraction protéique fortement enrichie en TGF-β selon l'invention.

### Fraction protéique fortement enrichie en TGF-β

Un autre objet de l'invention consiste en une fraction protéique fortement enrichie en TGF-β caractérisée en ce qu'elle comprend une teneur en TGF-β2, sous forme activée supérieure à 5 µg/g de protéines totales.

De préférence, la fraction protéique fortement enrichie en TGF-β, ci-dessus comprend une teneur en TGF-β2 comprise entre 6 µg et 15 µg de TGF-β2 sous forme activée, par gramme de protéines totales.

De préférence, la fraction fortement enrichie en TGFβ comprend une teneur en TGFβ2 sous forme activée, comprise entre 7 µg et 13µg, avantageusement entre 8 µg et 12 µg et de manière tout à fait préférée entre 9 µg et 11 µg de TGFβ2 par gramme de protéines totales.

La teneur en TGFβ2 de la fraction protéine enrichie selon l'invention peut être facilement déterminée par l'homme du métier, par exemple en mettant en oeuvre un test immunologique avec des anticorps spécifiques du TGFβ2, tel que celui qui est commercialisé par la Société R & D SYSTEMS (Barton Lane, Oxon, OX14345, Royaume-Uni) sous la marque QUANTIKINE™.

Le demandeur a montré que les protéines du lactosérum contenues dans la fraction protéique fortement enrichie en TGF-β sont majoritairement constituées d'α-lactalbumine et d'immunoglobulines.

Ainsi, la fraction protéique fortement enrichie en TGF-β selon l'invention contient entre 45 et 80% en poids d'α-lactalbumine et entre 10 et 25 % d'immunoglobulines, par rapport au poids total de la fraction.

De plus, cette fraction protéique enrichie en TGF-β est pratiquement exempte (moins de 11 %) de β-lactoglobuline, une protéine bien connue pour ses propriétés allergisantes.

La faible teneur en β-lactoglobuline dans la fraction protéique fortement enrichie en TGF-β selon l'invention la rend apte à une utilisation chez l'homme ou l'animal.

De plus, sans vouloir être lié par une quelconque théorie, le demandeur estime que la forte teneur en α-lactalbumine de la fraction protéique fortement enrichie en TGF-β selon l'invention peut renforcer significativement les propriétés thérapeutiques du TGF-β.

En effet, l'α-lactalbumine est riche en tryptophane, puisqu'elle contient quatre résidus de cet acide aminé par molécule de protéine. L'α-lactalbumine est la protéine du lait la plus riche en cet acide aminé. Le tryptophane est le précurseur de la sérotonine (5-hydroxytryptamine) et de la mélatonine . La sérotonine possède des propriétés sédatives et anti-stressogènes. La sérotonine diminue l'anxiété et favorise l'endormissement. Or, il est actuellement admis que le stress joue un rôle capital dans le développement du psoriasis. En effet, les patients atteints de psoriasis sont soumis à un stress permanent. La combinaison du TGF-β et de l'α-lactalbumine rend particulièrement apte la fraction protéique fortement enrichie en TGF-β selon l'invention à la fabrication de médicaments contre le psoriasis.

De plus, l'α-lactalbumine comprend un peptide allant de l'acide aminé en position 50 jusqu'à l'acide aminé en position 53, possédant des propriétés morphinomimétiques. Ce peptide opioïde issu de l'α-lactalbumine a été appelé « α-lactorphine » (voir H. CIBA and M. YOSHIKAWA, Biologically Functional Peptides from Food Proteins: New opioïdes peptides from milk proteins. Prot. Tail for Food and Med. uses, ed. M. DEKKA, N.Y., 1986, pp:123-153).

En outre, l'α-lactalbumine possède des propriétés bactéricides. Son hydrolyse dans le tractus intestinal par la trypsine et la chymotrypsine engendre la production de peptides bactéricides (voir A. PELLEGRINI et al., Isolation and Identification of Fluid Bactéricidal Domains in the bovine α-lactalbumine molecule, Biochim. Biophys. Acta, 1999, 1426:439-448). Les propriétés bactéricides de l'α-lactalbumine peuvent être bénéfiques chez des patients atteints de psoriasis. En effet, des agents pathogènes tels que les streptocoques et les staphylocoques, produisent des super-antigènes, qui sont des toxines bactériennes provoquant une activation et une hyperprolifération des lymphocytes T du sang, ce qui conduit un afflux des lymphocytes T activés au niveau de la peau. Il résulte de cet afflux une activation et une prolifération des kératinocytes, prolifération cellulaire à la base du psoriasis.

Sans vouloir être lié par une quelconque théorie, le demandeur pense que l'activité bactéricide de l'α-lactalbumine est de nature à limiter la prolifération des kératinocytes à la suite d'une infection bactérienne et en conséquence à potentialiser les effets anti-psoriasis du TGF-β.

En outre, la présence d'α-lactalbumine, en association avec le TGF-β, est de nature à accroître la stabilité du TGF-β dans une fraction protéique ou dans une composition selon l'invention, et notamment à protéger le TGF-β des diverses dégradations par les enzymes protéolytiques, par exemple lorsque la fraction ou la composition est administrée à un patient.

### Compositions pharmaceutiques selon l'invention.

Un autre objet de l'invention consiste en une composition pharmaceutique comprenant une fraction protéique fortement enrichie en TGF-β telle que définie ci-dessus, le cas échéant en association avec un ou plusieurs excipients physiologiquement compatibles.

Une composition pharmaceutique selon l'invention est en outre caractérisée en ce qu'elle contient une quantité thérapeutiquement efficace de TGF-β.

Selon une autre caractéristique, une composition pharmaceutique selon l'invention contient une quantité thérapeutiquement efficace d'une association entre le TGF-β et l'α-lactalbumine.

Lorsqu'elle est destinée à une administration topique, une composition pharmaceutique selon l'invention comprend de préférence de 1 nanogramme à 1 mg de TGF-β2 par dose, plus préférentiellement de 10 nanogrammes à 100 µg de TGF-β2 par dose et de manière tout à fait préférée de 50 nanogrammes à 20 µg par dose.

Lorsqu'elle est destinée à être administrée de manière systémique, une composition pharmaceutique selon l'invention est adaptée pour une administration quotidienne de 1 nanogramme à 500 µg de TGF-β2 par kg de poids du patient, de préférence 80 nanogrammes à 100 µg par kg de poids du patient et de manière tout à fait préférée de 1 µg à 20 µg par kg de poids du patient.

Une composition pharmaceutique selon l'invention peut se présenter sous la forme de comprimés, tablettes, gélules, sachets de poudre, solutions pour préparations injectables, lotions, pommades, crèmes dermiques, aérosols, onguents, bandages, émulsions, aérosols à utilisation intranasale ou bronchiale, implants, ou encore pâtes dentifrices ou bains de bouche.

Une composition pharmaceutique selon l'invention peut être appliquée par voie topique. Une telle composition pharmaceutique peut également être administrée localement ou par voie systémique.

En particulier, une composition pharmaceutique telle que définie ci-dessus peut être administrée par voie orale, entérale, parentérale, intradermique, sous-cutanée ou encore par voie intraveineuse.

Une composition pharmaceutique selon l'invention comprend, outre une quantité thérapeutiquement efficace de TGF-β2 ou d'une combinaison de TGF-β2 et d'α-lactalbumine, également des diluants, des conservateurs, des agents de solubilisation, des agents émulsifiants, des adjuvants ou des véhicules physiologiquement compatibles. De telles compositions peuvent se présenter sous forme liquide ou solide. Elles peuvent se présenter sous la forme de formulations lyophilisées ou de formulations de poudre sèche.

De telles compositions pharmaceutiques comprennent avantageusement des diluants tamponnés (p.ex. tris-HCl, acétate, phosphate), des additifs tels que l'albumine ou la gélatine afin d'empêcher leur absorption sur des surfaces, des détergents (p.ex. Tween 20, ™Tween 80, ™Pluronic S68™, sels d'acides biliaires, des agents de solubilisation (p.ex. alcool benzylique), des agents de charge ou des composés modificateurs de tonicité (p. ex. lactose, mannitol).

Une composition pharmaceutique selon l'invention peut également se présenter sous forme de formulations à base de particules de composés polymères tels que l'acide polylactique, d'acide polyglycolique, de polyvinylpyrrolidone, ou encore sous la forme de liposomes, de microémulsions, de micelles ou encore de vésicules unilamellaires ou multilamellaires.

De manière facultative, une composition pharmaceutique selon l'invention peut comprendre d'autres constituants : additifs tels que des antioxydants comme l'acide ascorbique, des polypeptides de faible poids moléculaire (moins de 10 acides aminés) tels des polyarginines. Elle peut également comprendre des acides aminés tels que la glycine, l'acide glutamique, l'acide aspartique ou l'arginine. Elle peut aussi comprendre des agents chélatants tels que l'EDTA. Elle peut aussi comprendre des sucres hydroxylés tels que le mannitol ou le sorbitol.

Pour des compositions pharmaceutiques à libération contrôlée ou à libération retardée, on ajoutera des acides gras, des cires ou des huiles. Font également partie de l'invention des compositions pharmaceutiques sous forme de particules dans lesquelles la fraction protéique enrichie en TGF-β est recouverte de polymères tels que les poloxamères ou les poloxamines.

Des compositions pour formulation topique englobent les gels, crèmes, solutions, émulsions, incluant des polymères de carbohydrates ou des matrices biodégradables de ceux-ci. Pour la réalisation d'une composition pharmaceutique selon l'invention, la fraction protéique fortement enrichie en TGF-β peut être encapsulée dans une cire, un film ou un véhicule solide, y compris une gomme à mâcher.

La fraction protéique fortement enrichie en TGF-β peut également être immobilisée afin d'empêcher sa dilution par la salive, par des agents tels que la méthyl-propylcellulose.

Une composition pharmaceutique selon l'invention possède de nombreuses autres applications cliniques dont certaines d'entre elles sont indiquées, de manière non limitative, ci-après.

Le TGF-β a de remarquables propriétés cicatrisantes. Une fraction protéique fortement enrichie en TGF-β selon l'invention peut être avantageusement appliquée dans le traitement de la cicatrisation des plaies externes ou internes, le cas échéant après intervention chirurgicale, ou encore dans le traitement des escarres ou encore dans la consolidation des os après fracture.

Une fraction protéique selon l'invention peut être également appliquée dans le traitement de l'ostéoporose, du fait des effets du TGF-β sur les ostéoclastes et les ostéoblastes, provoquant une induction de la formation du cartilage et de l'os.

Du fait de l'effet inhibiteur du TGF-β sur la prolifération des cellules. épithéliales, une fraction protéique fortement enrichie en TGF-β selon l'invention peuvent être avantageusement appliquée pour le traitement des mélanomes, des myélomes et des lymphomes.

La fraction protéique de l'invention peut être avantageusement utilisée en association avec la vitamine D3 et les rétinoïdes pour le traitement de certaines formes de leucémies. Elle peut être avantageusement appliquée pour lutter contre certaines formes de cancers, en particulier dans les cancers du sein.

De plus, la fraction protéique fortement enrichie en TGF-β selon l'invention peut également être utilisée pour la préparation d'un médicament destiné à la prévention ou au traitement de diverses maladies auto-immunes.

Elle peut aussi, être avantageusement appliquée à la prévention ou au traitement du lupus érythémateux, qui est une affection de la peau caractérisée par la présence de tâches ou plaques rouges recouvertes de squames, par une hyperkératose, par un processus inflammatoire lié à une infiltration et une activation des lymphocytes T dans le derme, et conduisant à la production d'auto-anticorps. Le lupus érythémateux est le prototype des maladies auto-immunes. L'administration d'une composition pharmaceutique selon l'invention aux patients atteints de lupus érythémateux, par voie orale, est susceptible d'améliorer significativement l'état général de ces malades.

Avantageusement, une fraction protéique fortement enrichie en TGF-β selon l'invention peut aussi être utilisée pour la préparation d'un médicament destiné à la prévention ou au traitement du psoriasis. Le psoriasis est une dermatose d'évolution chronique caractérisée par une hyperprolifération des kératinocytes associée à des anomalies de leur maturation. La prolifération des kératinocytes psoriasiques se manifeste par une augmentation du nombre des mitoses et un raccourcissement du cycle cellulaire. De plus, l'épiderme psoriasique est le siège d'un infiltrat inflammatoire dû à un afflux de lymphocytes T activés et à la libération de cytokines pro-inflammatoires. En raison de ces propriétés cytostatiques anti-inflammatoires et immunosuppressives, la fraction protéique fortement enrichie en TGF-β selon l'invention, lorsqu'elle est administrée par voie orale, constitue un traitement de fond du psoriasis.

Elle peut aussi constituer un traitement d'autres affections telles que l'arthrite rhumatismale, l'ostéoarthrite, la myasthénie grave, l'uvéite. On peut aussi envisager son utilisation lors de transplantations d'organes afin d'éviter, ou tout au moins réduire, les rejets de greffes.

Une fraction protéique fortement enrichie en TGF-β selon l'invention peut être également utilisée pour la fabrication d'un médicament destiné à la prévention ou au traitement de la maladie de Crohn. Cette affection est caractérisée par une inflammation de l'intestin, une altération de la régulation des antigènes du complexe majeur d'histocompatibilité (MHC) de classe II. L'inflammation intestinale est liée à une augmentation de l'expression des antigènes du MHC de classe II dans l'épithélium intestinal. Celle-ci est le résultat d'une attaque auto-immunitaire de l'intestin ou de perturbations de la régulation immunitaire.

Une telle composition pharmaceutique, lorsqu'elle est administrée par voie orale, constitue un traitement de fond de la maladie de Crohn.

L'invention a donc également pour objet l'utilisation d'une fraction protéique fortement enrichie en TGF-β telle que définie ci-dessus pour la préparation d'un médicament pour la prévention ou le traitement de maladies auto-immunes tels que le lupus érythémateux, le psoriasis, la maladie de Crohn, l'arthrite rhumatismale, l'ostéoarthrite, la myasténie grave ou l'uvéite.

Elle est également relative à l'utilisation d'une fraction protéique fortement enrichie en TGF-β pour la préparation d'un médicament destiné à la prévention ou au traitement des rejets de greffes.

L'invention concerne aussi l'utilisation d'une fraction protéique fortement enrichie en TGF-β telle que définie ci-dessus pour la préparation d'un médicament favorisant la cicatrisation.

Elle a également trait à l'utilisation d'une fraction protéique fortement enrichie en TGF-β selon l'invention pour la préparation d'un médicament destiné à la prévention ou au traitement de l'ostéoporose.

Selon un autre aspect, l'invention concerne l'utilisation d'une fraction protéique fortement enrichie en TGF-β selon l'invention pour la préparation d'un médicament oncostatique.

L'invention est en outre illustrée, sans pour autant être limitée par les exemples suivants:

### EXEMPLES.

### EXEMPLE 1:

### Préparation d'une solution riche en protéines du lactosérum (WPI) comme produit de départ du procédé selon l'invention.

10000 Kg de lait écrémé ayant une teneur en matière sèche de 92,9 g/Kg et une teneur en N x 6,38 de 35,4 g/kg étaient introduits, à 50°C, dans un équipement de microfiltration comportant 4,6 m² de membrane 0,1 µm Membralox® (alumine-zircone) et fonctionnant avec recirculation du microfiltrat à co-courant de manière à obtenir une pression transmembranaire uniforme égale à 0,6-0,7 bars.

La vitesse de balayage dans le compartiment Rétentat était fixée à 7 m/s.

Le débit d'extraction du microfiltrat était fixé à 345 l/h. Le débit d'extraction du rétentat était fixé à 172,5 l/h.

Les 6670 l de microfiltrat obtenus, ayant une teneur en matière sèche de 57,8 g/Kg et une teneur en N x 6,38 de 6,4 g/Kg étaient refroidis à 10 °C et introduits dans un équipement d'ultrafiltration comportant 9,6 m² de membrane Koch®, de conception spirale et ayant un pouvoir de coupure de 5 Kd. La pression de sortie était fixée à 3,4 bars.

Le débit d'extraction de l'ultrafiltrat était fixé à 250 l/h et celui du rétentat à 12,5 l/h. Les 334 l de rétentat étaient additionnés en continu de 1336 I d'eau osmosée jusqu'à élimination de ce volume d'eau ajoutée sous forme d'ultrafiltrat.

Le rétentat diafiltré final ainsi obtenu avait une teneur en protéines (N x 6,38) de 101,0 g/Kg et une teneur en matière sèche de 106,3 g/Kg. Il était soit utilisé immédiatement pour la préparation de la fraction enrichie en TGF-β, soit congelé à -30 °C.

### EXEMPLE 2:

### Préparation d'une fraction fortement enrichie en TGF-β à partir d'une solution riche en protéines du lactosérum.

200 Kg du rétentat diafiltré obtenu dans l'exemple 1, étaient dilués à 2000 I avec de l'eau osmosée à la température de 20°C dans une cuve équipée d'un agitateur à pales.

Après 10 minutes d'agitation, il était ajouté progressivement 1800 ml d'HCl 6N jusqu'à abaissement de la valeur du pH de 7,25 à 4,6. L'agitation était poursuivie pendant 10-15 minutes.

La solution était traitée thermiquement à 63°C-2 minutes et refroidie à 25 °C dans un équipement Actijoule® par aliquotes de 1000 I. Les 2000 I de suspension obtenus, portés à 35 °C, étaient ensuite microfiltrés, en continu, dans un équipement comportant 4,6 m² de membranes Sterilox® ayant un diamètre moyen de pores de 1,4 µm, avec recirculation à co-courant du microfiltrat de manière à obtenir une pression transmembranaire uniforme variant entre 0,4 et 0,8 bars, en 4h30.

Le débit d'extraction du microfiltrat était fixé à 400 l/h. Aucune extraction du rétentat n'était réalisée.

Lorsque le volume du rétentat était proche du volume mort de l'équipement de microfiltration, 320 l d'eau osmosée étaient ajoutés en continu et extraits sous forme de microfiltrat.

Les 53 Kg de rétentat diafiltré obtenus avaient une teneur en matière sèche de 39,96 g/Kg, une teneur en protéines ( N x 6,38 ) de 39,14 g/Kg et une teneur en TGF-β de 430,5 µg/Kg (soit 11 µg/g de protéine). Ils étaient congelés à -30°C et lyophilisés par aliquotes de 10 Kg.

### EXEMPLE 3:

### Analyse qualitative et quantitative de la fraction protéique fortement enrichie en TGF-β selon l'invention.

1 mg de la poudre lyophilisée obtenue à l'exemple 2 était dissout dans 1 ml d'eau milliQ puis diluée 5 fois dans le tampon A. L'équipement analytique utilisé était un chromatographe CLHP Waters 600 E équipé d'une colonne « source RPC 3 ml »(Pharmacia®).

Les deux tampons mis en oeuvre étaient :
Tampon A : Acide trifluoroacérique (TFA) 0.1 %
Tampon B : TFA 0.09 % dans de l'acétonitrile 90% 50 µl de produit étaient injectés et l'élution était réalisée par un gradient de 30 à 100 % de tampon B en 30 min au débit de 2 ml/min (à température ambiante). La détection était réalisée à 214 nm. Le traitement des aires chromatographiques était réalisé à l'aide du logiciel Nelson® ce qui permettait d'estimer les teneurs par rapport aux protéines totales : en α-lactalbumine 53 %, en serum-albumine 0,03 %, en β-lactoglobuline 10,9 %, en immunoglobulines 18,3 %.

La teneur en TGF-β2 des fractions enrichies était déterminée par une méthode d'immuno-essais utilisant des anticorps monoclonaux spécifiques du TGF-β2 (Kits Quantikine commercialisés par R & D Systems (Barton Lane, Oxon, OX14 3YS UK).

## Revendications

1. Procédé d'obtention d'une fraction protéique enrichie en TGFβ sous forme activée, à partir d'une solution riche en protéines solubles de la phase aqueuse du lait, ledit procédé comprenant les étapes suivantes :
a) ajustement des protéines solubles purifiées à une concentration de 5 à 30g/litre de solution ;
b) précipitation d'une partie des protéines du lactosérum par traitement acide de la solution à un pH compris entre 4 et 5,5 et à une température comprise entre 55°C et 68°C ;
c) microfiltration avec diafiltration, de la suspension précipitée afin d'obtenir respectivement un rétentat de microfiltration et un microfiltrat;
d) récupération du rétentat de microfiltration contenant la fraction protéique fortement enrichie en TGFβ ;
e) séchage du rétentat de microfiltration, diafiltré, afin d'obtenir une poudre fortement enrichie en TGFβ.

2. Procédé selon la revendication 1, **caractérisé en ce que** l'étape b) de précipitation est réalisée à un pH compris entre 4 et 5, et préférentiellement à un pH d'environ 4,6.

3. Procédé selon l'une des revendications 1 ou 2, **caractérisé en ce que** l'étape b) de précipitation est réalisée à une température comprise entre 55°C et 68°C et préférentiellement à environ. 63°C.

4. Procédé selon l'une des revendications 1 à 3, **caractérisé en ce que** la durée de l'étape b) de précipitation est comprise entre 30 secondes et 10 minutes, de préférence entre 30 secondes et 5 minutes, et de manière tout à fait préférée d'environ 2 minutes.

5. Procédé selon l'une des revendications 1 à 4, **caractérisé en ce qu'**à la fin de l'étape b), la solution liquide est refroidie rapidement.

6. Procédé selon l'une des revendications 1 à 5, **caractérisé en ce** la microfiltration de l'étape c) est réalisée sur une membrane ayant une taille moyenne de pores comprise entre 0,8 et 1,6 µm et possédant une distribution resserrée de tailles de pores et dans un équipement conduisant à l'obtention d'une pression transmembranaire uniforme.

7. Procédé selon l'une des revendications 1 à 6, **caractérisé en ce que** la diafiltration de l'étape b) est réalisée avec de l'eau osmosée ou de l'eau déminéralisée filtrée.

8. Procédé selon l'une des revendications 1 à 7, **caractérisé en ce que** la microfiltration avec diafiltration est réalisée à un pH compris entre 4 et 5,5, de préférence environ 4,6.

9. Procédé selon l'une des revendications 1 à 8, **caractérisé en ce que** la solution riche en protéines du lactosérum de départ est obtenue par la mise en oeuvre des étapes suivantes :
i) microfiltration tangentielle d'un lait écrémé sur une membrane de microfiltration ayant une taille moyenne de pores de 0,1 µm et dans un équipement permettant l'obtention d'une pression transmembranaire uniforme, puis récupération du microfiltrat ;
ii) concentration du rétentat obtenu à l'étape i) par ultrafiltration avec diafiltration ;
iii) récupération du rétentat d'ultrafiltration diafiltré.

10. Fraction protéique enrichie en TGFβ susceptible d'être obtenue par le procédé selon l'une des revendications 1 à 9, **caractérisée en ce qu'**elle comprend une teneur en TGFβ2 sous forme activée supérieure à 5 µg par gramme de protéines totales du lactosérum, et **en ce que** les autres protéines du lactosérum sont majoritairement constituées d'α-lactalbumine.

11. Fraction protéique selon la revendication 10 **caractérisée en ce qu'**elle contient de 45% à 80% en poids d'α-lactalbumine, par rapport au poids total de ladite fraction.

12. Composition pharmaceutique comprenant une fraction protéique selon l'une des revendications 10 et 11, le cas échéant en association avec un ou plusieurs excipients physiologiquement compatibles.

13. Utilisation d'une fraction protéique selon l'une des revendications 10 et 11 pour la préparation d'un médicament pour la prévention ou le traitement de maladies auto-immunes telles que le lupus érythémateux, le psoriasis ou la maladie de Crohn.

14. Utilisation d'une fraction protéique selon l'une des revendications 10 et 11 pour la préparation d'un médicament pour la prévention ou le traitement de l'arthrite rhumatismale, l'ostéoarthrite, la myasthénie ou l'uvéite.

15. Utilisation d'une fraction protéique selon l'une des revendications 10 et 11 pour la préparation d'un médicament favorisant la cicatrisation.

16. Utilisation d'une fraction protéique selon l'une des revendications 10 et 11 pour la préparation d'un médicament pour la prévention ou le traitement de l'ostéoporose.

17. Utilisation d'une fraction protéique selon l'une des revendications 10 et 11 pour la préparation d'un médicament oncostatique.

18. Utilisation d'une fraction protéique selon l'une des revendications 10 et 11 pour la préparation d'un médicament pour la prévention ou le traitement des rejets de greffe.

## Claims

1. A method for obtaining a TGFβ enriched protein fraction in activated form from a solution rich in soluble proteins from the milk aqueous phase, said method comprising the steps of:
a) adjusting the purified soluble proteins at a concentration between 5 and 30 g/liter of solution;
b) precipitating a part of the whey proteins by acid treatment of said solution at a pH ranging between 4 and 5.5 and at a temperature ranging between 55°C and 68°C;
c) carrying out a microfiltration with diafiltration of the precipitated suspension, so as to obtain respectively a microfiltration retentate and a microfiltrate;
d) recovering said microfiltration retentate comprising said protein fraction highly enriched in TGFβ ; and
e) drying said microfiltration retentate, diafiltrated, to obtain a powder highly enriched in TGFβ.

2. The method according to claim 1, **characterised in that** the precipitation step b) is performed at a pH comprised between 4 and 5, and preferably at a pH about 4.6.

3. The method according to one of claims 1 or 2, **characterised in that** the precipitation step b) is performed at a temperature comprised between 55°C and 68°C and preferably about 63°C.

4. The method according to one of claims 1 to 3, **characterised in that** the duration of the precipitation step b) is comprised between 30 seconds and 10 minutes, preferably between 30 seconds and 5 minutes, and more preferably about 2 minutes.

5. The method according to one of claims 1 to 4, **characterised in that** at the end of step b), the liquid solution is rapidly cooled down.

6. The method according to one of claims 1 to 5, **characterised in that** the microfiltration of step c) is performed using a membrane having an average pore size comprised between 0.8 and 1.6 µm and having a narrow range of pore size distribution and in a device leading to a uniform transmembrane pressure.

7. The method according to one of claims 1 to 6, **characterised in that** the diafiltration of step b) is performed using osmosis or demineralized filtrated water.

8. The method according to one of claims 1 to 7, **characterised in that** the microfiltration with diafiltration is carried out at a pH comprised between 4 and 5.5, and preferably about 4.6.

9. The method according to one of claims 1 to 8, **characterised in that** the starting solution rich in whey proteins is obtained by performing the following steps:
i) carrying out a cross-flow microfiltration of a skimmed milk on a microfiltration membrane having an average pore size of 0.1 µm and in a device allowing to obtain a uniform transmembrane pressure, then recovering the microfiltrate;
ii) concentrating the retentate obtained in step i) by ultrafiltration with diafiltration; and
iii) recovering the diafiltrated, ultrafiltration retentate.

10. ATGFβ enriched protein fraction liable to be obtained by the method according to one of claims 1 to 9, **characterised in that** it comprises a TGFβ2 content in an activated form higher than 5 µg per gram of total whey proteins and **in that** the other whey proteins consist mainly of α-lactalbumine.

11. The protein fraction according to claim 10, **characterised in that** it comprises weight % of α-lactalbumine based on the total weight of said fraction.

12. A pharmaceutical composition comprising a protein fraction according to one of claims 10 and 11, eventually in combination with one or several physiologically compatible excipients.

13. Use of a protein fraction according to one of claims 10 and 11 for the making of a drug for the prevention or treatment of auto-immune diseases such as erythematous lupus, psoriaris or Crohn disease.

14. Use of a protein fraction according to one of claims 10 and 11 for the making of a drug for the prevention or treatment of rheumatic arthristis, osteoarthritis, myasthenia or uveitis.

15. Use of a protein fraction according to one of claims 10 and 11 for the making of a drug enhancing healing.

16. Use of a protein fraction according to one of claims 10 and 11 for the making of a drug for the prevention or treatment of osteoporosis.

17. Use of a protein fraction according to one of claims 10 and 11 for the making of an oncostatic drug.

18. Use of a protein fraction according to one of claims 10 and 11 for the making of a drug for the prevention or treatment of graft rejections.

## Patentansprüche

1. Verfahren zur Gewinnung einer mit TGFβ in aktivierter Form angereicherten Proteinfraktion aus einer Lösung reich an löslichen Proteinen der wässerigen Phase der Milch, wobei das Verfahren die folgenden Schritte umfasst:
a) Anpassung der gereinigten löslichen Proteine an eine Konzentration von 5 bis 30 g/l Lösung;
b) Ausfällung eines Teils der Proteine des Laktoserums durch Säurebehandlung der Lösung bei einem pH-Wert zwischen 4 und 5,5 und bei einer Temperatur zwischen 55 °C und 68 °C;
c) Mikrofilterung mit Diafilterung der ausgefällten Suspension, um ein Mikrofiltrationsretentat bzw. ein Mikrofiltrat zu erhalten;
d) Wiedergewinnung des Mikrofiltrationsretentats, das die stark mit TGFβ angereicherte Proteinfraktion enthält;
e) Trocknen des diagefilterten Mikrofiltrationsretentats um ein stark mit TGFβ angereichertes Pulver zu erhalten.

2. Verfahren nach Anspruch 1, **dadurch gekennzeichnet, dass** der Schritt b) der Ausfällung bei einem pH-Wert zwischen 4 und 5 und vorzugsweise bei eine pH-Wert von ungefähr 4,6 durchgeführt wird.

3. Verfahren nach einem der Ansprüche 1 oder 2, **dadurch gekennzeichnet, dass** der Schritt b) der Ausfällung bei einer Temperatur zwischen 55 °C und 68 °C und vorzugsweise von ungefähr 63 °C durchgeführt wird.

4. Verfahren nach einem der Ansprüche 1 bis 3, **dadurch gekennzeichnet, dass** die Dauer des Schrittes b) der Ausfällung zwischen 30 Sekunden und 10 Minuten, vorzugsweise zwischen 30 Sekunden und 5 Minuten, und ganz vorteilhafterweise ungefähr 2 Minuten beträgt.

5. Verfahren nach einem der Ansprüche 1 bis 4, **dadurch gekennzeichnet, dass** am Ende des Schrittes b) die flüssige Lösung rasch abgekühlt wird.

6. Verfahren nach einem der Ansprüche 1 bis 5, **dadurch gekennzeichnet, dass** die Mikrofilterung des Schrittes c) auf einer Membran mit einer durchschnittlichen Porengröße zwischen 0,8 und 1,6 µm, die eine eingeschränkte Verteilung von Porengrößen besitzt, und in einer Ausrüstung erfolgt, die zum Erhalt eines einheitlichen Transmembrandrucks führt.

7. Verfahren nach einem der Ansprüche 1 bis 6, **dadurch gekennzeichnet, dass** die Diafilterung des Schrittes b) mit osmotisiertem Wasser oder gefiltertem entmineralisiertem Wasser erfolgt.

8. Verfahren nach einem der Ansprüche 1 bis 7, **dadurch gekennzeichnet, dass** die Mikrofilterung mit Diafilterung bei einem pH-Wert zwischen 4 und 5,5 und vorzugsweise von ungefähr 4,6 erfolgt.

9. Verfahren nach einem der Ansprüche 1 bis 8, **dadurch gekennzeichnet, dass** die Lösung reich an Proteinen des Ausgangslaktoserums durch den Einsatz der folgenden Schritte erhalten wird:
i) tangentiale Mikrofilterung einer entrahmten Milch auf einer Mikrofiltermembran mit einer durchschnittlichen Porengröße von 0,1 µm und in einer Ausrüstung, die den Erhalt eines einheitlichen Transmembrandrucks ermöglicht, dann Wiedergewinnung des Mikrofiltrats;
ii) Konzentration des in Schritt i) erhaltenen Retentats durch Ultrafilterung mit Diafilterung;
iii) Wiedergewinnung des diagefilterten Ultrafilterungsretentats.

10. Mit TGFβ angereicherte Proteinfraktion, die durch das Verfahren nach einem der Ansprüche 1 bis 9 gewonnen werden kann, **dadurch gekennzeichnet, dass** sie einen Gehalt an TGFβ in aktivierter Form von mehr als 5 µg pro Gramm von Gesamtproteinen des Laktoserums umfasst, und dass die anderen Proteine des Laktoserums mehrheitlich von α-Laktalbumin gebildet sind.

11. Proteinfraktion nach Anspruch 10, **dadurch gekennzeichnet, dass** sie 45 Gew.-% bis 80 Gew.-% a-Laktalbumin bezogen auf das Gesamtgewicht der Fraktion enthält.

12. Pharmazeutische Zusammensetzung, umfassend eine Proteinfraktion nach einem der Ansprüche 10 und 11, gegebenenfalls in Verbindung mit einem oder mehreren physiologisch kompatiblen Exzipienten.

13. Verwendung einer Proteinfraktion nach einem der Ansprüche 10 und 11 1 für die Herstellung eines Medikaments zur Prävention oder Behandlung von Autoimmunerkrankungen, wie beispielsweise Lupus erythematodes, Psoriasis oder Crohn-Krankheit.

14. Verwendung einer Proteinfraktion nach einem der Ansprüche 10 und 11 für die Gewinnung eines Medikaments zur Prävention oder Behandlung von rheumatoider Arthritis, Osteoarthritis, Myasthenie oder Uveitis.

15. Verwendung einer Proteinfraktion nach einem der Ansprüche 10 und 11 für die Gewinnung eines Medikaments, das die Narbenbildung begünstigt.

16. Verwendung einer Proteinfraktion nach einem der Ansprüche 10 und 11 für die Gewinnung eines Medikaments zur Prävention oder Behandlung der Osteoporose.

17. Verwendung einer Proteinfraktion nach einem der Ansprüche 10 und 11 für die Gewinnung eines onkostatischen Medikaments.

18. Verwendung einer Proteinfraktion nach einem der Ansprüche 10 und 11 für die Gewinnung eines Medikaments zur Prävention oder Behandlung der Abstoßung von Transplantaten.
